# EUROPEAN PATENT APPLICATION

(11) **EP 4 722 366 A1**
(43) Date of publication of application: **08.04.2026**
(21) Application number: 24814553.4
(22) Date of filing: 30.05.2024
(51) Int. Cl.: C12N 15/113, C12N 15/861, C12N 5/10, A61P 27/16, A61K 48/00

(54) **METHOD FOR PERFORMING GENE THERAPY AND REGENERATION OF COCHLEAR SUPPORTING CELLS**

(30) Priority: 02.06.2023 CN 202310650073
(71) Applicant: Otovia Therapeutics, Suzhou, Jiangsu 215000 (CN)
(72) Inventor: CHAI, Renjie, Nanjing, Jiangsu 210024 (CN); CUI, Zhiping, Shanghai 201210 (CN); SONG, Huaien, Shanghai 201210 (CN); ZHANG, Shanzhong, Shanghai 201210 (CN); SUN, Sijie, Shanghai 201210 (CN); ZHANG, Liyan, Nanjing, Jiangsu 210024 (CN); SUN, Qiuhan, Nanjing, Jiangsu 210024 (CN); QI, Jieyu, Nanjing, Jiangsu 210024 (CN); TAN, Fangzhi, Shanghai 201210 (CN); TAN, Chang, Shanghai 201210 (CN); LU, Ling, Nanjing, Jiangsu 210008 (CN)
(74) Representative: Nederlandsch Octrooibureau
(86) International application number: PCT/CN2024/096420
(87) International publication number: WO 2024/245357

(57) **Abstract**

A method for performing gene therapy and regeneration of cochlear supporting cells. Specifically, provided is a cochlear-supporting-cell-specific promoter as shown in SEQ ID NO: 1, comprising: (1) an enhancer sequence as shown in SEQ ID NO: 2 or a sequence having at least 90% sequence identity thereto and retaining the function of SEQ ID NO: 2; and (2) a core promoter element SEQ ID NO: 3. The promoter specifically drives the expression of a transgene in the cochlear supporting cells.

## Description

### TECHNICAL FIELD

The present invention relates to genetic engineering technology, and in particular to a method for performing gene therapy and regeneration of cochlear supporting cells.

### BACKGROUND

Hearing loss is the fourth leading factor affecting the lifespan of people with disabilities worldwide, affecting 6% to 8% of the population and severely reducing their quality of life. In recent years, efforts to treat hearing loss have increasingly focused on gene therapy, which may serve as a solution for treating hearing loss. Some deafness genes are expressed in cochlear supporting cells (SCs), so gene therapy targeting inner ear SCs is very necessary. For example, epidemiological studies have shown that a large proportion of cases of hereditary hearing loss (30-50% in various ethnic groups) are caused by loss-of-function mutations in a single gene GJB2, which encodes a major cochlear gap junction protein (GJ), connexin 26 (Cx26), mainly expressed in SCs. Therefore, if this protein can be specifically re-expressed in SCs, it is expected to restore hearing loss caused by GJB2 deficiency, which will provide a theoretical basis for the clinical treatment of this hereditary hearing loss. However, specific gene therapy methods for SCs remain scarce. It is necessary to search for new gene therapy methods targeting SCs to treat hereditary sensorineural hearing loss caused by GJB2 deficiency.

However, the vectors commonly used in current inner ear gene therapy to promote the expression of specific transgenes in cochlear cells are generally broad-spectrum and cannot target specific cell types.

### SUMMARY

To address the deficiencies of the prior art, the present invention provides polynucleotides comprising the promoter region of cochlear supporting cells (SCs), and vectors comprising the region, which can be used to promote the expression of specific transgenes in cochlear supporting cells (SCs).

The present invention provides a cochlear supporting cell-specific enhancer, comprising the sequence shown in SEQ ID NO: 2 or a homologous sequence having at least 90% sequence identity thereto and retaining the function of SEQ ID NO: 2. The homologous sequence is a sequence derived from a mammal, preferably a primate, and more preferably Hominidae.

The present invention further provides a cochlear supporting cell-specific promoter, comprising:
(1) the sequence shown in SEQ ID NO: 2 or a sequence having at least 90% sequence identity thereto and retaining the function of SEQ ID NO: 2; and
(2) a core promoter element.

In one or more embodiments, the core promoter element (2) is a promoter suitable for initiating gene expression in mammalian cells.

In one or more embodiments, the core promoter element (2) is the SHH gene promoter.

In one or more embodiments, the sequence of the core promoter element (2) is shown in SEQ ID NO: 3 or a homologous sequence having at least 90% sequence identity thereto. The homologous sequence is the promoter sequence of the SHH gene derived from a mammal, preferably a primate, and more preferably Hominidae.

The present invention further provides a nucleic acid construct, comprising the cochlear supporting cell-specific promoter described in any one of the embodiments herein.

In one or more embodiments, the nucleic acid construct further comprises a gene of interest, which is operably linked to the cochlear supporting cell-specific promoter.

In one or more embodiments, the gene is selected from the group consisting of a deafness gene, a transcription factor gene, a signaling pathway gene, and a tool gene for manipulating genes in cochlear supporting cells or expressed in cochlear supporting cells.

In one or more embodiments, the nucleic acid construct further comprises a multiple cloning site and/or a terminator. Preferably, the terminator is a polyA terminator.

In one or more embodiments, the nucleic acid construct is a vector, such as a cloning vector, an integrating vector, or an expression vector. In one or more embodiments, the vector is an AAV vector.

The present invention further provides an rAAV vector system, comprising an AAV vector and a nucleic acid construct encoding an AAV virus-required gene, wherein the AAV vector comprises the cochlear supporting cell-specific promoter described in any one of the embodiments herein.

In one or more embodiments, the AAV vector further comprises a gene of interest, which is operably linked to the cochlear supporting cell-specific promoter.

In one or more embodiments, the AAV virus-required gene comprises one or more selected from the group consisting of Rep, Cap, E2A, E4, and VA genes.

The present invention further provides an rAAV viral particle, comprising the cochlear supporting cell-specific promoter described in any one of the embodiments herein.

The present invention further provides a host cell comprising or having integrated into its genome: the cochlear supporting cell-specific promoter, nucleic acid construct, or AAV vector system described in any one of the embodiments herein.

The present invention further provides a pharmaceutical composition, comprising pharmaceutically acceptable excipients, and one or more selected from the group consisting of the cochlear supporting cell-specific promoter, nucleic acid construct, rAAV vector system, rAAV viral particles, and host cell described in any one of the embodiments herein.

The present invention further provides an in vitro method for expressing a gene in cochlear supporting cells, comprising the step of introducing a nucleic acid construct comprising the gene into cochlear supporting cells, wherein the gene is operably linked to the cochlear supporting cell-specific promoter described in any one of the embodiments herein.

In one or more embodiments, the gene is selected from the group consisting of deafness genes, transcription factor genes, signaling pathway genes, and tool genes for manipulating genes in cochlear supporting cells or expressed in cochlear supporting cells. The deafness genes are those expressed in cochlear supporting cells, including GJB2, GJB3, GJB6, MYH14, WFS1, EYA4, TMPRSS3, DCDC2, GPSM2, and KARS. The transcription factor genes are those associated with hair cell regeneration, comprising ATOH1, POU4F3, GFI1, IKZF2, INSM1, and SIX1. The signaling pathway genes are those in the Wnt, Notch, Hippo, and TGF-beta signaling pathways associated with hair cell regeneration.

The present invention further provides the use of the sequence shown in SEQ ID NO: 2, or a sequence having at least 90% sequence identity thereto and retaining the function of SEQ ID NO: 2, in a chimeric promoter.

The present invention further provides a method for preparing a chimeric promoter, comprising the step of operably linking (1) the sequence set forth in SEQ ID NO: 2, or a sequence having at least 90% sequence identity thereto and retaining the function of SEQ ID NO: 2, with (2) a core promoter element.

The present invention further provides the use of the cochlear supporting cell-specific promoter described in any embodiment herein in preparing a medicament for treating diseases caused by genetic defects in cochlear supporting cells, wherein the medicament is used to express the gene in cochlear supporting cells.

The gene is selected from the group consisting of deafness genes, transcription factor genes, signaling pathway genes, and tool genes for manipulating genes in cochlear supporting cells or expressed in cochlear supporting cells. The deafness genes are those expressed in cochlear supporting cells, including GJB2, GJB3, GJB6, MYH14, WFS1, EYA4, TMPRSS3, DCDC2, GPSM2, and KARS. The transcription factor genes are those associated with hair cell regeneration, comprising ATOH1, POU4F3, GFI1, IKZF2, INSM1, and SIX1. The signaling pathway genes are those in the Wnt, Notch, Hippo, and TGF-beta signaling pathways.

In one or more embodiments, the disease is hereditary deafness caused by genetic defects in cochlear supporting cells. Preferably, the disease is hereditary deafness caused by the loss-of-function of GJB2.

The present invention further provides a method for treating diseases caused by genetic defects in cochlear supporting cells, comprising the step of expressing the defective gene in cochlear supporting cells, wherein the gene is operably linked to the cochlear supporting cell-specific promoter described in any one of the embodiments herein.

In one or more embodiments, the method comprises the step of introducing a nucleic acid construct or vector comprising the coding sequence of the gene into cochlear supporting cells, wherein the coding sequence of the gene is operably linked to the cochlear supporting cell-specific promoter.

In one or more embodiments, the gene is a deafness gene expressed in cochlear supporting cells, including GJB2, GJB3, GJB6, MYH14, WFS1, EYA4, TMPRSS3, DCDC2, GPSM2, and KARS.

In one or more embodiments, the disease is hereditary deafness caused by the loss-of-function of GJB2.

The present invention further provides a method for inducing cochlear supporting cells to differentiate into hair cells, comprising the step of expressing transcription factor genes and/or signaling pathway genes associated with hair cell regeneration in cochlear supporting cells, wherein the genes are operably linked to the cochlear supporting cell-specific promoter described in any embodiment herein. The method is an in vitro method.

In one or more embodiments, the method comprises the step of introducing a nucleic acid construct or vector containing the coding sequences of the transcription factor and/or signaling pathway genes into cochlear supporting cells, wherein the coding sequences are operably linked to the cochlear supporting cell-specific promoter.

In one or more embodiments, the transcription factor genes comprise ATOH1, POU4F3, GFI1, IKZF2, INSM1, and SIX1.

In one or more embodiments, the signaling pathway genes are those in the Wnt, Notch, Hippo, and TGF-beta signaling pathways associated with hair cell regeneration.

### Beneficial Effects of the Present Invention

After injecting control group viruses AAV-ie-CAG-nls-EGFP and AAV-ie-CAG-nls-EGFP-mGJB2 into the cochleae of neonatal mice, ABR audiometry was performed on day 30. In the experimental group, hearing impairment was observed after the CAG promoter drove GJB2 expression in SCs and hair cells (HCs). This indicates that gene expression driven by broad-spectrum promoters such as CAG is not suitable for the gene therapy of deafness caused by GJB2 mutations.

By injecting virus AAV-Sox47-mNeonGreen (Sox47 promoter sequence: SEQ ID NO: 1) into the cochleae of neonatal mice and performing immunofluorescence staining on the 14th day, it was found that the green fluorescent protein mNeonGreen was specifically expressed in SCs. Furthermore, the cochlear supporting cell-specific promoter of the present invention has a therapeutic effect on hereditary deafness. In addition, the introduction of exogenous genes is safe, avoiding gene overexpression in hair cells that would otherwise cause damage to the hair cells.

### BRIEF DESCRIPTION OF THE DRAWINGS

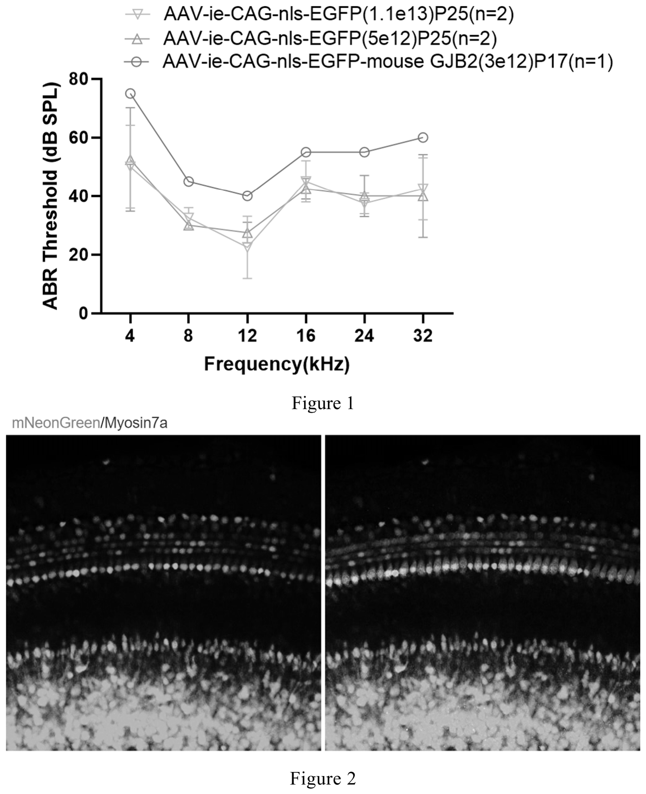

Fig. 1 shows the results of ABR performed on day 30 after injecting control viruses AAV-ie-CAG-nls-EGFP and AAV-ie-CAG-nls-EGFP-mouse GJB2 into the cochleae of neonatal mice. In the experimental group, hearing impairment was observed after the CAG promoter drove GJB2 expression in the regions of cochlear supporting cells (SCs) and hair cells (HCs). This indicates that gene expression driven by broad-spectrum promoters such as CAG is not suitable for the gene therapy of deafness caused by GJB2 mutations.
Fig. 2 shows that after injecting the virus AAV-ie-CAG-mNeonGreen into the cochleae of neonatal mice, cochleae were collected for immunofluorescence staining on day 14. The green fluorescent protein mNeonGreen was expressed in both hair cells (HCs) and cochlear supporting cells (SCs), and fluorescent expression was also observed in other regions. This indicates that the CAG promoter is widely expressed in various cells of the cochlea without specificity.

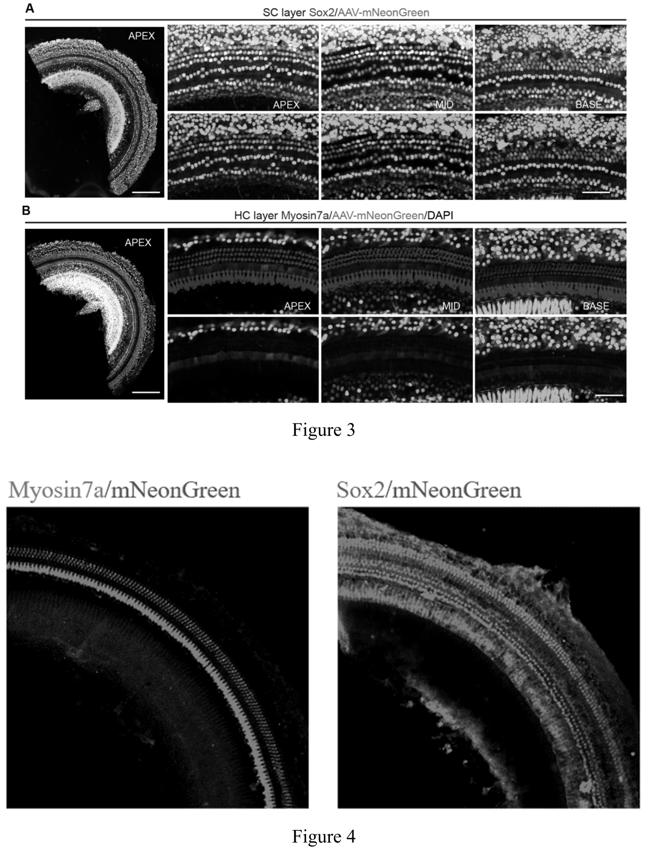

Fig. 3 shows the results of immunofluorescence staining for the Sox47 promoter. After injecting the virus AAV-ie-Sox47-mNeonGreen (Sox47 sequence: SEQ ID NO: 1) into the cochleae of neonatal mice, cochleae were collected for immunofluorescence staining on day 14. The results show that the Sox47 promoter drives the specific expression of the mNeonGreen reporter gene in cochlear supporting cells (SCs).
Fig. 4 shows the results of immunofluorescence staining for the SHH gene promoter. After injecting the virus AAV-ie-SHH-mNeonGreen (SHH sequence: SEQ ID NO: 3) into the cochleae of neonatal mice, cochleae were collected for immunofluorescence staining on day 14. The results show that the mNeonGreen reporter gene was not expressed in either hair cells or cochlear supporting cells of the cochlea. This indicates that the SHH promoter itself cannot drive the specific expression of the transgene in cochlear supporting cells.

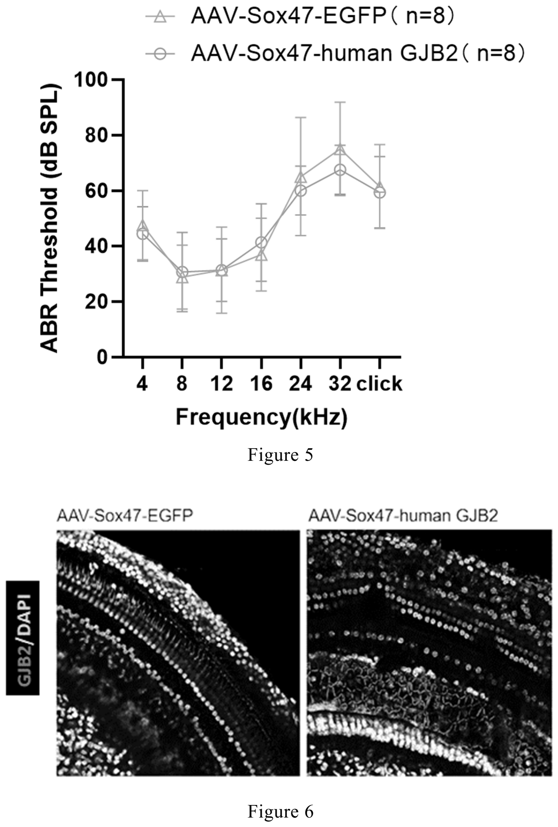

Fig. 5 shows the results of ABR audiometry performed after injecting EGFP or GJB2 driven by the AAV-Sox47 promoter into the cochleae of mice. The results demonstrate that, unlike the CAG promoter-driven GJB2 in Figure 1 which caused hearing impairment, the GJB2 driven by the Sox47 promoter did not affect the hearing of mice.
Fig. 6 shows the results of restored expression of GJB2 driven by the Sox47 promoter in SCs of GJB2 knockout mice. Compared with the KO mice, the expression of GJB2 was restored after Sox47-GJB2 injection.

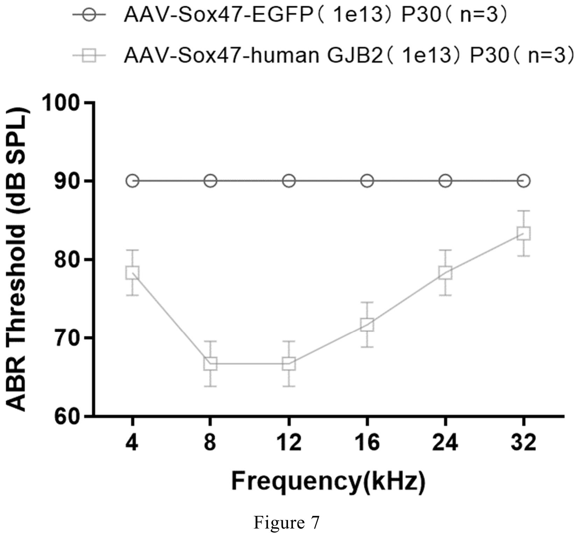

Fig. 7 shows the results of ABR audiometry for mouse hearing after the re-expression of GJB2 driven by the Sox47 promoter. Compared with the KO mice, the hearing of the KO mice was restored to a certain level after Sox47-GJB2 injection.

### DETAILED DESCRIPTION

Unless otherwise defined, the embodiments of the present invention will employ conventional techniques of molecular biology (including recombinant technology), microbiology, cell biology, biochemistry, and immunology, all of which are within the skill of the art. These techniques are fully described in the literature, such as Molecular Cloning: A Laboratory Manual, Second Edition (Sambrook et al., 1989); Oligonucleotide Synthesis (M.J. Gait, ed., 1984); Animal Cell Culture (R.I. Freshney, ed., 1987); Methods in Enzymology (Academic Press, Inc.); Current Protocols in Molecular Biology (F.M. Ausubel et al., eds., 1987 and its regular updates); PCR: The Polymerase Chain Reaction (Mullis et al., eds., 1994); A Practical Guide to Molecular Cloning (Perbal Bernard V., 1988); Phage Display: A Laboratory Manual (Barbas et al., 2001).

### Specific Promoter

The inventors have discovered an enhancer capable of specifically enhancing the transcriptional activity of the core promoter element in cochlear supporting cells, which has the sequence shown in SEQ ID NO: 2 or a homologous sequence having at least 90% sequence identity thereto. This enhancer can be used to construct a chimeric promoter. Based on this, compared with the prior art, the present invention provides a cochlear supporting cell-specific promoter and a therapeutic method for specifically targeting cochlear SCs: by means of the promoter capable of specifically targeting cochlear SCs, a viral vector containing the promoter delivers a specific gene to SCs in a targeted manner, promoting the expression of the transgene in SCs, thereby: 1. performing gene therapy for hearing loss caused by deafness gene mutations; 2. achieving the regeneration of SCs into hair cells by introducing exogenous genes to treat hereditary deafness, and the method is safe, avoiding gene overexpression in hair cells that would otherwise cause damage to the hair cells.

A promoter is a DNA sequence recognized, bound, and transcribed by RNA polymerase, located upstream of the start codon of structural genes (usually within about 100 to 1000 bp) that control transcription. It comprises conserved sequences required for RNA polymerase-specific binding and transcription initiation, and the promoter itself is not transcribed. Its general structure includes a core promoter element and upstream regulatory elements. The core promoter element further comprises a transcription start site (TSS) and a TATA box, which primarily serves as the binding site for RNA polymerase and the site where transcription is initiated. The upstream regulatory elements can alter transcription efficiency by binding to their corresponding trans-acting factors.

In some embodiments, the cochlear supporting cell-specific promoter of the present invention comprises: (1) the sequence shown in SEQ ID NO: 2, or a homologous sequence having at least 90% sequence identity thereto and retaining the function of SEQ ID NO: 2; and (2) the core promoter element shown in SEQ ID NO: 3. The present invention further provides a method for preparing a chimeric promoter, comprising the step of operably linking the aforementioned (1) with (2). In one or more embodiments, (1) is located at the 5' end of (2).

Herein, the core promoter element comprised in the specific promoter refers to any promoter suitable for initiating gene expression in mammalian cells. In one or more embodiments, the core promoter element described in (2) is a promoter that does not drive gene expression in cochlear hair cells (HCs). Those skilled in the art can easily obtain the sequences of these promoters. In exemplary embodiments, the core promoter element is a promoter of a mammalian, preferably human, SHH gene. The promoter of the human SHH gene is shown in SEQ ID NO: 3.

In other embodiments, the cochlear supporting cell-specific promoter of the present invention is SOX47 (SEQ ID NO: 1) or a sequence having at least 90% sequence identity thereto.

The present invention further relates to homologous sequences having at least 50%, at least 70%, at least 80%, at least 90%, at least 95%, at least 98%, or at least 99% sequence identity to each of the aforementioned nucleic acid sequences (e.g., the sequences of each component in the specific promoter). Herein, a "homologous sequence" refers to a sequence derived from a species that belongs to the same order, family, genus, or species as the species of the source sequence, and has the aforementioned sequence identity.

The present invention further relates to a polynucleotide that hybridizes to the sequence of the aforementioned specific promoter and has at least 50%, preferably at least 70%, more preferably at least 80% sequence identity thereto. The present invention particularly relates to a polynucleotide that can hybridize with the polynucleotide of the present invention under stringent conditions. As used herein, "stringent conditions" refer to: (1) hybridization and elution under conditions of low ionic strength and high temperature, such as 0.2×SSC, 0.1% SDS, 60°C; (2) hybridization in the presence of a denaturant, such as 50% (v/v) formamide, 0.1% fetal bovine serum/0.1% Ficoll, 42°C, etc.; or (3) hybridization only occurs when the sequence identity between the two sequences is at least 90%, more preferably at least 95%.

### Nucleic Acid Construct

The present invention further provides a nucleic acid construct comprising the aforementioned specific promoter, which is used for genetic functions such as gene cloning or expression. The nucleic acid construct may be an expression cassette or a vector. In addition to the specific promoter, the nucleic acid construct may further comprise other elements required for cloning or expression, such as a multiple cloning site (MCS) and/or a terminator (e.g., a polyA signal sequence). For example, the nucleic acid construct may be an RNA construct or a DNA vector capable of transfecting cells and driving gene expression. As used herein, the terms "nucleic acid molecule", "polynucleotide", and "nucleic acid" are used interchangeably.

To express a polypeptide or protein, the nucleic acid construct further comprises a gene of interest, which is operably linked to the specific promoter. As used herein, a gene refers to a coding sequence capable of encoding a polypeptide or protein. In some embodiments of the present invention, the gene is selected from the group consisting of deafness genes, transcription factor genes, signaling pathway genes, and various tool genes for manipulating genes in cochlear supporting cells or expressed in cochlear supporting cells.

As used herein, "deafness genes" refer to deafness-related genes expressed in cochlear supporting cells, including but not limited to GJB2, GJB3, GJB6, MYH14, WFS1, EYA4, TMPRSS3, DCDC2, GPSM2, and KARS. As used herein, "transcription factor genes" refer to transcription factor genes associated with hair cell regeneration, including but not limited to ATOH1, POU4F3, GFI1, IKZF2, INSM1, and SIX1. As used herein, "signaling pathway genes" refer to genes in the Wnt, Notch, Hippo, and TGF-beta signaling pathways that are associated with hair cell regeneration.

Where the gene or its sequence is known, the coding nucleic acid of each gene can be prepared by conventional methods in the art, and the corresponding vector can be constructed. Methods well known to those skilled in the art can be used to construct the recombinant vector, see, for example, the techniques described in Sambrook et al. (2001, Molecular Cloning: A Laboratory Manual, Cold Spring Harbor Laboratory), Ausubel et al. (1989, Short Protocols in Molecular Biology, Wiley) or other standard textbooks. Alternatively, the nucleic acid construct can be reconstituted into liposomes for delivery to target cells. The nucleic acid construct of the present invention can be transferred into host cells by well-known methods, which varies depending on the type of host cell. For example, calcium chloride transfection is commonly used for prokaryotic cells, while calcium phosphate treatment or electroporation can be used for other host cells, see Sambrook et al (as mentioned above).

The vectors described herein comprise the nucleic acid molecules or expression cassettes described herein. Vectors may be plasmids, cosmids, viruses, or viral vectors. Vectors include cloning vectors, integrating vectors, and may also be expression vectors. Expression vectors typically comprise sequences for plasmid maintenance and for cloning and expressing exogenous nucleotide sequences. The sequences (referred to collectively as "flanking sequences" in certain embodiments) generally comprise one or more of the following nucleotide sequences: a promoter (e.g., the specific promoter described herein), one or more enhancer sequences, an origin of replication, a transcription termination sequence, complete intron sequences containing donor and acceptor splice sites, sequences encoding a leader sequence for polypeptide secretion, a ribosome-binding site, a polyadenylation sequence, a multiple cloning site (MCS) for inserting a nucleic acid encoding the protein to be expressed, and a selectable marker element. Typically, the promoter is located 5' to the multiple cloning site or the polypeptide-encoding sequence. In one or more embodiments, the expression vector uses an AAV series vector as the backbone.

The vector may optionally comprise a "tag" coding sequence, i.e., an oligonucleotide molecule located at the 5' or 3' end of the polypeptide-encoding sequence; the oligonucleotide sequence encodes poly-histidine (such as 6His) or another "tag", such as FLAG, HA, or myc. This tag is typically fused to the polypeptide when the polypeptide is expressed, and may serve as a means for affinity purification of the protein from host cells or for detecting the protein. Affinity purification can be achieved, for example, by column chromatography using an antibody against the tag as the affinity matrix. The tag may optionally be subsequently removed from the purified protein by various means, such as using certain peptidases for cleavage.

The flanking sequences may be homologous (i.e., derived from the same species and/or strain as the host cell), heterologous (i.e., derived from a species other than the host cell's species or strain), hybrid (i.e., a combination of flanking sequences from more than one source), synthetic, or natural. Similarly, the source of the flanking sequences may be any prokaryotic or eukaryotic organism, any vertebrate or invertebrate organism, or any plant, provided that the flanking sequences function in and are activatable by the host cell machinery.

The selectable marker gene encodes a protein necessary for the survival and growth of host cells grown in a selective medium. Typical selectable marker genes encode proteins that: (a) confer resistance to antibiotics or other toxins (e.g., ampicillin, tetracycline, or kanamycin for prokaryotic host cells); (b) complement the auxotrophy of the cell; or (c) provide an essential nutrient not obtainable from a complex or defined medium. Specific selectable markers include the kanamycin resistance gene, the ampicillin resistance gene, and the tetracycline resistance gene. Beneficially, the neomycin resistance gene can also be used for selection in both prokaryotic and eukaryotic host cells.

### AAV Vectors and AAV Particles

As used herein, the term "adeno-associated virus" or "AAV" includes members of the viral group associated with this name, which belongs to the genus Dependoparvovirus and the family Parvoviridae. AAV is a single-stranded DNA virus that replicates only in cells where certain functions are provided by a co-infecting helper virus. General information and reviews on AAV can be seen, for example, Carter (1989, Handbook of Parvoviruses, Volume 1, pages 169-228). Multiple serotypes of the virus are known to be suitable for gene delivery, and the various serotypes are closely related structurally and functionally. All AAV serotypes apparently exhibit highly similar replication characteristics mediated by homologous rep genes, and all encode three related capsid proteins. At least 13 sequentially numbered AAV serotypes are known in the art. Non-limiting exemplary serotypes used in the methods disclosed herein include any of the 13 serotypes, such as AAV2, AAV8, and AAV9. An AAV particle comprises, consists essentially of, or consists of the following three major viral proteins: VP1, VP2, and VP3. In one or more embodiments, AAV refers to serotypes AAV1, AAV2, AAV4, AAV5, AAV6, AAV7, AAV8, AAV9, AAV10, AAV11, AAV12, AAV13, AAVPHP.B, or AAVrh74.

In some embodiments, the nucleic acid construct of the present invention is an AAV vector, which expresses the gene of interest via a recombinant AAV (rAAV) vector system. As used herein, an "AAV vector" refers to a vector that comprises, consists essentially of, or consists of the specific promoter described herein and one or more AAV inverted terminal repeats (ITRs). When present in a host cell that provides functional rep and cap gene products, such an AAV vector can be replicated and packaged into infectious viral particles; e.g., by transfecting the host cell. In one or more embodiments, the AAV vector comprises the specific promoter described herein, at least one nucleic acid encoding at least one protein or RNA, and/or enhancers and/or terminators flanked by ITRs for packaging into infectious AAV particles. Plasmids comprising the AAV vector may also comprise elements for production purposes, such as antibiotic resistance genes and the like.

The present invention further provides a recombinant AAV (rAAV) vector system, comprising the AAV vector and other nucleic acid constructs encoding genes required for AAV virus production, wherein the AAV vector comprises the cochlear supporting cell-specific promoter described in any one of the embodiments herein. In one or more embodiments, the AAV vector further comprises the gene of interest described herein, which is operably linked to the cochlear supporting cell-specific promoter. In one or more embodiments, the AAV vector system comprises an Anc80L65 plasmid, a helper plasmid, and the aforementioned AAV vector.

As used herein, the term "viral capsid" or "capsid" refers to the protein coat or capsid of a viral particle. The function of the capsid is to encapsidate, protect, and transport the viral genome, as well as release it into the host cell. The capsid is typically composed of oligomeric structural subunits of proteins ("capsid proteins"). As used herein, the term "encapsidation" refers to being enclosed within a viral capsid. The viral capsid of AAV is composed of a mixture of three viral capsid proteins: VP1, VP2, and VP3.

The term "AAV viral particle" or "AAV particle" refers to a viral particle that consists of at least one AAV capsid protein and an encapsidated polynucleotide AAV vector. Thus, the production of AAV particles necessarily involves the production of AAV vectors, as such vectors are contained within the AAV particles. The present invention provides a recombinant AAV (rAAV) viral particle, which comprises the specific promoter described herein and optionally further comprises a gene of interest operably linked to the specific promoter. In one or more embodiments, the rAAV viral particle is selected from one or more of rAAV1, rAAV2, rAAV4, rAAV5, rAAV6, rAAV7, rAAV8, rAAV9, rAAV10, rAAV11, rAAV12, rAAV13, rAAVPHP.B, or rAAVrh74.

As used herein, the term "helper" regarding viruses or plasmids refers to a virus or plasmid used to provide additional components required for the replication and packaging of any of the AAV vectors disclosed herein. Components encoded by a helper virus may include any genes necessary for virion assembly, encapsidation, genomic replication, and/or packaging. For example, a helper virus or plasmid may encode enzymes required for viral genome replication. Non-limiting examples of helper viruses and plasmids suitable for use with AAV constructs include pHELPer (plasmid), adenovirus (virus), or herpesvirus (virus).

Methods for producing and using recombinant AAV (rAAV) vectors are known in the art. Methods for producing rAAV particles and heterologous nucleic acids are also known in the art and commercially available (see, e.g., US 2007/0015238 and US 2012/0322861, the entire contents of which are incorporated herein by reference). For example, a plasmid comprising a heterologous nucleic acid sequence can be combined with one or more helper plasmids (e.g., those comprising rep genes (e.g., encoding Rep78, Rep68, Rep52, and Rep40) and cap genes (encoding VP1, VP2, and/or VP3)), and transfected into or stably integrated into a producer cell line, such that rAAV particles can be packaged and subsequently purified.

In some embodiments, one or more helper plasmids comprise a first helper plasmid comprising rep genes and cap genes, and a second helper plasmid comprising E1a, E1b, E4, E2a, and VA genes. In some embodiments, the rep gene is derived from AAV2, and the cap gene is derived from AAV44.9. Helper plasmids and methods for preparing such plasmids are known in the art and commercially available.

### Host Cell

The present invention further includes host cells that comprise or have integrated into their genome the specific promoter or nucleic acid construct described herein. Host cells can serve as recipients for vectors. A host cell may be "transfected" or "transformed," which refers to the process by which exogenous nucleic acid is transfected or transduced into the host cell. Transformed cells include primary target cells and their progeny. As used herein, the terms "engineered" and "recombinant" cells or host cells generally refer to cells into which an exogenous nucleic acid sequence, such as a vector, has been introduced. Thus, recombinant cells can be distinguished from naturally occurring cells that do not comprise the introduced recombinant nucleic acid.

Suitable host cells, as described herein, include, but are not limited to, Chinese Hamster Ovary (CHO) cells, HeLa cells, Baby Hamster Kidney (BHK) cells, Monkey Kidney (COS) cells, human hepatocellular carcinoma cells (e.g., HepG2), cochlear supporting cells and the like.

As used herein, a "packaging cell (or helper cell)" refers to a cell used for producing viral vectors. The production of recombinant AAV viral vectors requires trans-provided Rep and Cap proteins, as well as gene sequences from adenoviruses that assist in AAV replication. In some aspects, the packaging/helper cell comprises plasmids stably integrated into the cell genome. In other aspects, the packaging cell may be transiently transfected. Typically, the packaging cell is a eukaryotic cell, such as a mammalian cell or an insect cell. In one or more embodiments, the cell used for producing the viral vector is an HEK293 cell, including HEK293T, HEK293H, HEK293F, HEK293S, HEK293T/17, HEK293T/17SF, HEK293FT, HEK293SG, HEK293E, HEK293-6E, HEK293FTM, and HEK293SGGD cells.

The nucleic acid construct/recombinant expression vector of the present invention can be introduced into cells of interest. Methods for introducing the same are conventional in the art, including but not limited to viral transduction, microinjection, particle bombardment, gene gun transformation, electroporation, and the like. In certain embodiments, electroporation is employed to introduce the nucleic acid construct or recombinant expression vector into said cells. When the host cells are cultured under appropriate conditions, they synthesize the fusion protein, which can then be collected from the culture medium (if the host cells secrete it into the culture medium) or directly from the host cells producing it (if not secreted).

### Methods for Preparing AAV Vectors and Viral Particles

Various methods can be used for preparing AAV viral vectors. In one or more embodiments, packaging is achieved by using helper viruses or helper plasmids and a cell line. Helper viruses or helper plasmids comprise elements and sequences that facilitate the production of viral vectors. In some embodiments, the helper plasmids are stably integrated into the genome of the packaging cell line, such that the packaging cell line does not require additional transfection with the helper plasmids.

In one or more embodiments, the cell is a packaging cell line or a helper cell line. In one or more embodiments, the helper cell line is a eukaryotic cell; for example, HEK 293 cells or 293T cells. In one or more embodiments, the helper cell is a yeast cell or an insect cell.

Helper plasmids may comprise, for example, at least one viral helper DNA sequence which is derived from a replication-defective viral genome encoding trans full virion proteins required for packaging replication-defective AAV, and which is used to generate virion proteins capable of packaging replication-defective AAV at high titer without producing replication-competent AAV.

Helper plasmids for packaging AAV are known in the art (see, e.g., U.S. Patent Publication No. 2004/0235174 A1, the entire contents of which are incorporated herein by reference). As described therein, by way of non-limiting example, an AAV helper plasmid may comprise Ad5 genes E2A, E4, and VA as helper viral DNA sequences, which are controlled by their respective native promoters or heterologous promoters. The AAV helper plasmid may further comprise an expression cassette for expressing a marker protein (e.g., a fluorescent protein) to facilitate easy detection of the transfection of desired target cells.

Provided herein are methods for producing AAV particles, comprising transfecting a packaging cell line with any of the AAV helper plasmids disclosed herein; and any of the AAV vectors disclosed herein. In some embodiments, the AAV helper plasmid and the AAV vector are co-transfected into the packaging cell line. In some embodiments, the cell line is a mammalian cell line, e.g., a human embryonic kidney (HEK) 293 cell line. Provided herein are cells comprising any of the AAV vectors and/or AAV particles disclosed herein.

### Pharmaceutical Composition

The present invention further provides a pharmaceutical composition comprising pharmaceutically acceptable excipients, and one or more selected from the group consisting of the specific promoter, nucleic acid construct, rAAV vector system, rAAV viral particles, and host cells described herein. In one or more embodiments, the pharmaceutical composition comprises an AAV vector and pharmaceutically acceptable excipients, wherein the AAV vector comprises the specific promoter described herein operably linked to a gene of interest. In one or more embodiments, the pharmaceutical composition comprises rAAV viral particles and pharmaceutically acceptable excipients, wherein the rAAV viral particles comprise a polynucleotide that comprises the specific promoter described herein operably linked to a gene of interest.

As used herein, the term "pharmaceutically acceptable excipients" encompasses any standard pharmaceutical carriers, such as phosphate-buffered saline solutions, water, and emulsions (e.g., oil/water or water/oil emulsions), and various types of wetting agents. The composition may further comprise stabilizers and preservatives. For examples of carriers, stabilizers, and adjuvants, see Martin (1975) Remington's Pharm. Sci., 15th Edition (Mack Publishing Company, Easton).

As described herein, the pharmaceutical composition can be formulated by any method known or developed in the field of pharmacology, including but not limited to contacting the active ingredient (e.g., viral particles or recombinant vectors) with excipients or other auxiliary components, and dividing or packaging the product into dosage units. The viral particles herein can be formulated to have desired characteristics, such as enhanced stability, improved cell transfection, sustained or delayed release, favorable biodistribution or tropism, regulated or enhanced translation of the encoded protein in vivo, and an optimized release profile of the encoded protein in vivo.

Thus, the pharmaceutical composition may further comprise saline, lipids, liposomes, lipid nanoparticles, polymers, lipoplexes, core-shell nanoparticles, peptides, proteins, cells transfected with viral vectors (e.g., for transplantation into a subject), nanoparticle mimetics, or combinations thereof. In some embodiments, the pharmaceutical composition is formulated as nanoparticles. In some embodiments, the nanoparticles are self-assembled nucleic acid nanoparticles.

Pharmaceutical compositions according to the present invention may be prepared, packaged, and/or sold as a single unit dose and/or as multiple single unit doses. The amount of the active ingredient is typically equal to the dose of the active ingredient to be administered to a subject and/or an appropriate fraction of such a dose (e.g., one-half or one-third of such a dose). The formulations of the present invention may include one or more excipients, where the amounts of each excipient together enhance the stability of the viral vector, improve cell transfection or viral vector transduction, increase the expression of the protein encoded by the viral vector, and/or alter the release profile of the protein encoded by the viral vector. In some embodiments, the pharmaceutical composition comprises an excipient. Non-limiting examples of excipients include solvents, dispersion media, diluents, or other liquid carriers, dispensing or suspending aids, surfactants, isotonic agents, thickeners, or emulsifiers, preservatives, or combinations thereof.

In some embodiments, the pharmaceutical composition comprises a cryoprotectant. As used herein, the term "cryoprotectant" refers to an agent capable of reducing or eliminating damage to the substance during freezing. Non-limiting examples of cryoprotectants include sucrose, trehalose, lactose, glycerol, dextrose, raffinose, and/or mannitol.

### Use and Methods

The present invention provides the use of the sequence shown in SEQ ID NO: 2, or a homologous sequence having at least 90% sequence identity thereto and retaining the function of SEQ ID NO: 2, in a chimeric promoter. Furthermore, there is also provided the use of the specific promoter in the preparation of a medicament for treating diseases caused by gene defects in cochlear supporting cells, wherein the medicament is used for expressing the defective gene in cochlear supporting cells.

The present invention provides a method of introducing a target gene into cells of a subject, comprising contacting the cells with an effective amount of any of the AAV viral particles disclosed herein, wherein the particles comprise a polynucleotide that includes the specific promoter described herein operably linked to a gene of interest.

The present invention provides a method of expressing a gene of interest in cochlear supporting cells, comprising the step of introducing a nucleic acid construct comprising the gene into cochlear supporting cells, wherein the gene is operably linked to the specific promoter described herein. Typically, the promoter is located in the same expression cassette as the gene and upstream of the gene. Examples of the gene of interest are as described elsewhere herein.

The specific promoter described herein is operably linked to a therapeutic gene, providing a theoretical basis for the clinical targeted therapy of hereditary hearing loss. The present invention provides a method of treating diseases caused by gene defects in cochlear supporting cells, comprising the step of expressing the defective gene in cochlear supporting cells, wherein the gene is operably linked to the cochlear supporting cell-specific promoter described herein. The method further comprises the step of introducing a nucleic acid construct or vector comprising the coding sequence of the gene into cochlear supporting cells, wherein the coding sequence of the gene is operably linked to the cochlear supporting cell-specific promoter. Exemplarily, the gene is a deafness gene expressed in cochlear supporting cells, including GJB2, GJB3, GJB6, MYH14, WFS1, EYA4, TMPRSS3, DCDC2, GPSM2, and KARS. In some embodiments, the gene is GJB2. In one or more embodiments, the disease is hereditary deafness caused by the loss-of-function of GJB2.

The present invention further provides a method of inducing cochlear supporting cells to differentiate into hair cells, comprising the step of expressing transcription factors and/or signaling pathway genes associated with hair cell regeneration in cochlear supporting cells, wherein the genes are operably linked to the specific promoter described herein. Specifically, the method comprises the step of introducing a nucleic acid construct or vector comprising the coding sequences of the transcription factors and/or signaling pathway genes into cochlear supporting cells, wherein the coding sequences are operably linked to the cochlear supporting cell-specific promoter. Exemplarily, the transcription factor genes include ATOH1, POU4F3, GFI1, IKZF2, INSM1, and SIX1, and the signaling pathway genes are those in the Wnt, Notch, Hippo, and TGF-beta signaling pathways associated with hair cell regeneration.

As used herein, the term "disease and/or condition" refers to the physical state of a subject associated with the disease and/or condition described herein. As used herein, "treatment" includes any beneficial or desired effect on the symptoms or lesions of a disease or pathological condition, and may include even a minimal reduction in one or more measurable markers of the disease or condition being treated (e.g., hereditary deafness). Treatment may optionally include reduction or alleviation of the symptoms of the disease or condition, or delay in the progression of the disease or condition. "Treatment" does not necessarily mean complete eradication or cure of the disease or condition or its associated symptoms. As used herein, "treatment" of a disease in a subject refers to (1) preventing the occurrence of symptoms or the disease in a subject who is susceptible to the disease or has not yet exhibited symptoms of the disease; (2) inhibiting the disease or arresting its development; (3) ameliorating or causing regression of the disease or its symptoms. As understood in the art, "treatment" is a method for achieving beneficial or desired results, including clinical results. Beneficial or desired results may include one or more, but are not limited to, alleviation or improvement of one or more symptoms (whether detectable or undetectable), reduction in the severity of the condition (including the disease), a stabilized (i.e., not worsening) state of the condition (including the disease), delay or slowing of the progression of the condition (including the disease), improvement or mitigation of the condition (including the disease), and remission (whether partial or complete).

The term "subject" or "patient" may refer to cells or animals (e.g., mammals or humans) administered the pharmaceutical composition of the present invention for treating, preventing, ameliorating, and/or alleviating the disease or condition of the present invention. The subject is not limited to a specific species, and includes non-human animals undergoing diagnosis or treatment, and those used as infected animals or animal models, including but not limited to dogs, monkeys, cattle, horses, apes, mice, rats, canines, and rabbit species, as well as other livestock, sport animals, or companion animals. In some embodiments, the subject is a human.

As used herein, the term "effective amount" is intended to refer to an amount sufficient to achieve the desired effect. In the context of therapeutic or prophylactic uses, the effective amount will depend on the type and severity of the condition in question, as well as the characteristics of the individual subject (e.g., general health, age, sex, body weight, and tolerance to the pharmaceutical composition). In the context of gene therapy, in some embodiments, the effective amount is an amount sufficient to result in the restoration of partial or full function of the defective gene in the subject. In other embodiments, the effective amount of the AAV viral particles is an amount sufficient to result in the expression of the gene in the subject. In some embodiments, the effective amount is the amount required to increase galactose metabolism in a subject in need thereof. Those skilled in the art will be able to determine the appropriate amount based on these and other factors.

In some embodiments, the effective amount will depend on the size and nature of the use. It also depends on the nature and sensitivity of the target subject, as well as the method of administration. Those skilled in the art will be able to determine the effective amount based on these and other considerations. According to embodiments, the effective amount may comprise one or more administrations of the composition, consist essentially of one or more administrations of the composition, or consist of one or more administrations of the composition.

As used herein, the term "administration" is intended to refer to delivering a substance to a subject, such as an animal or a human. Administration may be carried out as a single dose, continuously, or intermittently during the entire course of treatment. Methods for determining the most effective method and dosage of administration are known to those skilled in the art, and will vary depending on the composition used for treatment, the purpose of treatment, and the age, health, or sex of the subject being treated. Single or multiple administrations may be performed, with the dosage level and regimen selected by the treating physician, or, in the case of companion animals and other animals, by the treating veterinarian.

### Dosage and Administration

Methods for determining the most effective method of administration and dosage are known in the art, and will vary depending on the composition used for treatment, the purpose of treatment, and the subject being treated. Single or multiple administrations may be performed, with the dosage level and regimen selected by the treating physician. The dosage may be affected by the route of administration. Suitable dosage formulations and methods for administering the agent are known in the art. Non-limiting examples of such suitable dosages may range from as low as 10E9 vector genomes to as high as 10E17 vector genomes per administration.

In embodiments of the methods described herein, the number of viral particles (e.g., AAV) administered to a subject ranges from about 10E9 to about 10E17. In certain specific embodiments, about 10E10 to about 10E12, about 10E11 to about 10E13, about 10E11 to about 10E12, about 10E11 to about 10E14, about 5×10E11 to about 5×10E12, or about 10E12 to about 10E13 viral particles are administered to a subject.

In some embodiments, the AAV particles repair the genetic defect in a subject. In some embodiments, in a successfully treated cell, tissue, organ, or subject, the ratio of the repaired target polynucleotide (e.g., GJB2) or polypeptide to the unrepaired target polynucleotide or polypeptide is at least about 1.5:1, about 2:1, about 3:1, about 4:1, about 5:1, about 6:1, about 7:1, about 8:1, about 9:1, about 10:1, about 20:1, about 50:1, about 100:1, about 1000:1, about 10,000:1, about 100,000:1, or about 1,000,000:1. The amount or ratio of the repaired target polynucleotide or polypeptide can be determined by any method known in the art, including but not limited to Western blotting, Northern blotting, Southern blotting, PCR, sequencing, mass spectrometry, flow cytometry, immunohistochemistry, immunofluorescence, fluorescence in situ hybridization (FISH), sequencing, immunoblotting, and ELISA.

In some embodiments, the viral particles are administered to a subject via intravenous, intrathecal, intracerebral, intraventricular, intranasal, intratracheal, intraauricular, intraocular or periocular, oral, rectal, transmucosal, inhalation, transdermal, parenteral, subcutaneous, intradermal, intramuscular, intrapleural, topical, intralymphatic, or intracisternal administration; such administration may also be via intraarterial, intracardiac, subventricular, epidural, intracerebral, intraventricular, subretinal, intravitreal, intraarticular, intraperitoneal, intrauterine, or any combination thereof. In some embodiments, the delivery of the viral particles is systemic.

The administration of the AAV vectors, AAV particles, or compositions of the present disclosure may be performed as a single dose, continuously, or intermittently during the entire course of treatment. In some embodiments, the AAV vectors, AAV particles, or compositions of the present disclosure are administered parenterally via injection, infusion, or implantation. The AAV particles and compositions of the present disclosure may be administered in combination with other known therapies for the condition being treated.

### Kit

In some embodiments, the specific promoters, nucleic acid constructs, cells, viral particles, or pharmaceutical compositions described herein may be assembled into a therapeutic, diagnostic, or research kit to facilitate their use in therapeutic, diagnostic, or research applications. In some embodiments, the kits of the present disclosure comprise any one of the AAV vectors, AAV particles, cells, or pharmaceutical compositions described herein.

In some embodiments, the kit further comprises instructions for use. Specifically, such a kit may include one or more reagents described herein, as well as instructions describing the intended applications and proper use of these reagents. In some embodiments, the kit may comprise instructions regarding mixing one or more components of the kit and/or isolating and mixing a sample and administering it to a subject. In some embodiments, the reagents in the kit are pharmaceutical formulations and dosages suitable for the specific application and the administration method of the reagents. Kits for research purposes may comprise components at appropriate concentrations or amounts for performing various experiments.

The kit can be designed to facilitate the use of the methods described herein and can take a variety of forms. Where applicable, each composition of the kit may be provided in a liquid form (e.g., in a solution) or a solid form (e.g., a lyophilized powder). In certain cases, some compositions may be configurable or otherwise processable (e.g., processed into an active form), for example, by adding a suitable solvent or other substance (e.g., water or cell culture medium), which may or may not be provided with the kit. In some embodiments, the composition may be provided in a preservation solution (e.g., a cryopreservation solution). Non-limiting examples of preservation solutions include DMSO and paraformaldehyde. In some embodiments, the preservation solution comprises an amount of a metalloproteinase inhibitor.

In some embodiments, the kit comprises any one or more of the components described herein in one or more containers. Thus, in some embodiments, the kit may include a container holding the reagents described herein. The reagents may be in the form of a liquid, gel, or solid (powder). The reagents may be sterilely prepared, packaged in syringes, and shipped frozen. Alternatively, they may be contained in vials or other containers for storage. A second container may contain other sterilely prepared reagents.

Alternatively, the kit may comprise premixed active agents and be shipped in syringes, vials, tubes, or other containers. The kit may have one or more or all of the components necessary for administering the reagents to a subject, such as syringes, topical application devices, or IV catheters and bags.

### Partial Specific Embodiment Items

1. A cochlear supporting cell-specific enhancer, comprising the sequence shown in SEQ ID NO: 2, or a homologous sequence having at least 90% sequence identity thereto and retaining the function of SEQ ID NO: 2;
   preferably, the homologous sequence is a sequence derived from a mammal, preferably derived from a primate, and more preferably derived from Hominidae.
2. A cochlear supporting cell-specific promoter, comprising:
   (1) the sequence shown in SEQ ID NO: 2, or a sequence having at least 90% sequence identity thereto and retaining the function of SEQ ID NO: 2; and
   (2) a core promoter element;

   preferably, the core promoter element (2) is the promoter of the SHH gene;
   preferably, the sequence of the core promoter element (2) is shown in SEQ ID NO: 3, or a homologous sequence having at least 90% sequence identity thereto;
   more preferably, the homologous sequence is a promoter sequence of the SHH gene derived from a mammal, preferably a derived from primate, and more derived from preferably Hominidae.
3. A nucleic acid construct, comprising the cochlear supporting cell-specific promoter according to Item 2;
   preferably, the nucleic acid construct further comprises a gene of interest, which is operably linked to the cochlear supporting cell-specific promoter; and/or the nucleic acid construct is a vector;
   more preferably, the gene of interest is selected from the group consisting of a deafness gene, a transcription factor gene, a signaling pathway gene, a tool gene for manipulating genes in cochlear supporting cells, or a tool gene expressed in cochlear supporting cells.
4. A recombinant AAV (rAAV) vector system, comprising an AAV vector and a nucleic acid construct encoding an AAV virus-required gene, wherein the AAV vector comprises the cochlear supporting cell-specific promoter according to Item 2;
   preferably, the AAV vector further comprises a gene of interest, which is operably linked to the cochlear supporting cell-specific promoter.
5. A recombinant AAV (rAAV) virion, comprising the cochlear supporting cell-specific promoter according to Item 2.
6. A host cell, comprising or having integrated into its genome: the cochlear supporting cell-specific promoter according to Item 2, the nucleic acid construct according to Item 3, or the AAV vector system according to Item 4.
7. A pharmaceutical composition, comprising a pharmaceutically acceptable excipient, and one or more selected from the group consisting of: the cochlear supporting cell-specific promoter according to Item 2, the nucleic acid construct according to Item 3, the AAV vector system according to Item 4, the recombinant AAV (rAAV) virion according to Item 5, or the host cell according to Item 6.
8. A method for expressing a gene in cochlear supporting cells, comprising the step of introducing a nucleic acid construct comprising the gene into cochlear supporting cells, wherein the gene is operably linked to the cochlear supporting cell-specific promoter according to Item 2.
9. Use of the sequence shown in SEQ ID NO: 2, or a homologous sequence having at least 90% sequence identity thereto and retaining the function of SEQ ID NO: 2, in a chimeric promoter.
10. A method for preparing a chimeric promoter, comprising the step of operably linking (1) the sequence shown in SEQ ID NO: 2, or a sequence having at least 90% sequence identity thereto and retaining the function of SEQ ID NO: 2, with (2) a core promoter element.
11. Use of the cochlear supporting cell-specific promoter according to Item 2 in preparing a medicament for treating a disease caused by a genetic defect in cochlear supporting cells, wherein the medicament is used for expressing the defective gene in cochlear supporting cells,
   preferably, the disease is hereditary deafness caused by a genetic defect in cochlear supporting cells,
   more preferably, the disease is hereditary deafness caused by a loss-of-function of GJB2.
12. A method for inducing cochlear supporting cells to differentiate into hair cells, comprising the step of expressing transcription factors and/or signaling pathway genes associated with hair cell regeneration in cochlear supporting cells, wherein the genes are operably linked to the cochlear supporting cell-specific promoter according to Item 2;
   preferably, the method comprises the step of introducing a nucleic acid construct or vector containing the coding sequences of the transcription factors and/or signaling pathway genes into cochlear supporting cells, wherein the coding sequences are operably linked to the cochlear supporting cell-specific promoter.

The present invention will be illustrated below by specific examples. It should be understood that these examples are merely illustrative and are not intended to limit the scope of the present invention. The methods and materials used in the examples are conventional methods and materials in the art unless otherwise specified.

### Examples

### Example 1: Effect of CAG Promoter-Driven GJB2 Expression in SCs and HCs on Hearing

### 1.1 Construction of Plasmids AAV-CAG-nls-EGFP (SEQ ID NO: 6) and AAV-CAG-nls-EGFP-mouse GJB2 (SEQ ID NO: 7), and Viral Packaging

The constructed plasmids AAV-CAG-nls-EGFP and AAV-CAG-nls-EGFP-mouse GJB2 (mouse GJB2 gene, NCBI Accession No.: 14619) were co-transfected into HEK-293T cells together with the AAV-ie capsid protein (Reference: DOI: 10.1038/s41467-019-11687-8) and the pHelper plasmid (NCBI Accession No.: AF369965.1) in appropriate amounts. lodixanol gradient centrifugation was used for purifying the AAV virions, and the viral titer was measured to be 1E12^{~}1E13 GC/mL.

### 1.2 Hearing Loss Induced by CAG Promoter-Driven GJB2 Expression in the Cochlea

Neonatal FVB mice at postnatal day 2 (P2) were injected with the viruses AAV-ie-CAG-nls-EGFP and AAV-ie-CAG-nls-EGFP-mouse GJB2 via round window injection. One month later, auditory brainstem response (ABR) audiometry was performed, and hearing loss was observed in the AAV-ie-CAG-nls-EGFP-mouse GJB2 group (Fig. 1). These results indicate that the non-specific cellular expression of the CAG ubiquitous promoter is not conducive to targeted gene therapy. It is because GJB2 is mainly expressed in supporting cells (SCs), and if overexpressed in hair cells (HCs), it may lead to hearing impairment.

### 1.3 Expression of mNeonGreen in SCs and HCs Driven by CAG Promoter

Neonatal FVB mice at postnatal day 2 (P2) were injected with AAV-ie-CAG-mNeonGreen via round window injection. The cochleae were harvested for immunofluorescence staining after seven days, the hair cell marker myosin7a was used for staining hair cells, and DAPI was used for nuclear staining. It was observed that mNeonGreen was expressed in both SCs and HCs regions (Fig. 2).

### Example 2: Enhancement of Transgene Expression in SCs by Sox47 Promoter

### 2.1 Construction of Plasmid AAV-Sox47-mNeonGreen (SEQ ID NO: 8) and Viral Packaging

The Sox47-specific promoter (SEQ ID NO: 1) was constructed into the pAAV-mNeonGreen plasmid. Specifically, the vector was obtained by digesting the pAAV-mNeonGreen plasmid; subsequently, the target fragment of the Sox47-specific promoter (SEQ ID NO: 1) was synthesized via gene synthesis, and the complete target plasmid was constructed through recombination. mNeonGreen (DNA sequence: SEQ ID NO: 4; amino acid sequence: SEQ ID NO: 5) is a green fluorescent protein used to indicate infected and expressing cells, similar to the green fluorescent protein EGFP. The constructed viral packaging plasmid AAV-Sox47-mNeonGreen was co-transfected into HEK-293T cells together with the AAV-ie capsid protein and the pHelper plasmid in appropriate amounts. AAV virions were purified by iodixanol gradient centrifugation, and the viral titer was measured to be 1E12^{~}1E13 GC/mL.

### 2.2 Targeted Driving Effect of Sox47 Promoter on mNeonGreen in SCs

Neonatal FVB mice at postnatal day 2 were injected with the virus AAV-ie-Sox47-mNeonGreen via round window injection. On day 15, the cochleae were harvested, and immunofluorescence staining was performed on the mouse cochleae using the hair cell marker myosin7a and the supporting cell (SC) marker Sox2. It was observed that mNeonGreen was specifically expressed in the SCs region, as shown in Fig. 3 (Scale bars: 200 µm; 50 µm).

The two AAV-ie-Sox47-mNeonGreen viruses comprising the specific promoter were injected into the inner ears of mice at postnatal days 2-3 (P2-P3) via round window administration. The mice were anesthetized at low temperature on ice for 1-2 minutes. After anesthesia, a postauricular incision was made to expose the round window, and the cochlea was visualized prior to manual injection using a glass micropipette. Following injection, the skin incision was sealed with 3M Vetbond tissue adhesive. The injected mice were then placed on a 37°C heating pad for recovery. Neonatal mice fully recovered within approximately 10 minutes after operation and were returned to their dams. Standard postoperative care was provided following the operation. Two weeks later, the infection efficiency of the viruses on cochlear hair cells was evaluated, and data were statistically analyzed. Cochleae of mice were subjected to immunofluorescence staining with the antibody against the hair cell marker Myo7a, the antibody against the supporting cell marker gene Sox2, and the nuclear marker DAPI. Before performing cochlear surface preparation, the cochleae were fixed and decalcified. After being dissected and the tectorial membrane removed, the samples were washed three times with PBS and blocked with blocking buffer for 1 hour. Subsequently, the samples were incubated overnight at 4°C with primary antibody (Myo7a, 1:1000). The tissues were then washed three times with PBS and counterstained with secondary antibody (Donkey anti-rabbit Alexa Fluor 555, 1:4000) and DAPI (1:1000) at room temperature for 1 hour, followed by three additional washes with PBS. Slides were retrieved and labeled with mouse information and cochlear processing procedures. Anti-fade mounting medium was added dropwise to the slides, and coverslips were gently placed to avoid air bubbles. The slides were stored in a cool and dark place. Confocal images were acquired using a Zeiss LSM700 laser scanning confocal microscope. Complete cochlear images were processed with ImageJ software, and Z-stack projections were generated along the Z-axis using the same software. Double-positive cells for Myo7a-labeled hair cells (HCs) and mNeonGreen were counted. The immunofluorescence staining results demonstrated that the mNeonGreen reporter gene was exclusively and specifically expressed in cochlear supporting cells (SCs) (Fig. 3).

### Example 3: The SHH Promoter Sequence Alone (SEQ ID NO: 3) Does Not Drive Gene Expression in Cochlear Cells

### 3.1 Plasmid Construction and Viral Packaging

The SHH promoter (SEQ ID NO: 3) was constructed into the pAAV-mNeonGreen plasmid to generate the AAV-SHH-mNeonGreen viral packaging plasmid. This plasmid was co-transfected into HEK-293T cells in an appropriate amount together with the AAV-ie capsid protein plasmid and the pHelper plasmid. AAV virions were purified by iodixanol gradient centrifugation, and the viral titer was determined to be 1E12^{~}1E13 genome copies (GC)/mL, which was regarded as an appropriate concentration.

### 3.2 The SHH Promoter Alone (SEQ ID NO: 3) Does Not Drive mNeonGreen Expression in Cochlear Cells

Via round window administration, the aforementioned AAV-ie-SHH-mNeonGreen virus containing the specific promoter was injected into the inner ears of mice at P2-P3. The mice were anesthetized at low temperature on ice for 1-2 minutes. After anesthesia, a postauricular incision was made to expose the round window, and the cochlea was visualized prior to manual injection using a glass micropipette. Following injection, the skin incision was sealed with 3M Vetbond tissue adhesive. Two weeks later, the infection efficiency of the virus on cochlear hair cells (HCs) was evaluated, and data were statistically analyzed. Cochleae of mice were subjected to immunofluorescence staining with the hair cell marker myosin7a and the nuclear marker DAPI. Before performing cochlear surface preparation, the cochleae were fixed and decalcified. After being dissected and the tectorial membrane removed, the samples were washed three times with PBS and blocked with blocking buffer for 1 hour. Subsequently, the samples were incubated overnight at 4°C with primary antibody (Myo7a, 1:1000). The tissues were then washed three times with PBS and counterstained with secondary antibody (Donkey anti-rabbit Alexa Fluor 555, 1:4000) and DAPI (1:1000) at room temperature for 1 hour. Double-positive cells for Myo7a-labeled HCs and mNeonGreen were counted. The immunofluorescence staining results demonstrated that the mNeonGreen reporter gene was not expressed in either cochlear HCs or supporting cells (SCs) (Fig. 4). The Sox47 promoter (SEQ ID NO: 1) consists of two components: an enhancer sequence (SEQ ID NO: 2) and the core SHH promoter sequence (SEQ ID NO: 3). Results from Examples 1 and 3 demonstrated that the specificity of the Sox47 promoter is determined by the enhancer sequence (SEQ ID NO: 2).

### Example 4: Effects of Sox47 Promoter-Driven GJB2 Expression in SCs on Hearing

### 4.1 AAV-Sox47-EGFP and AAV-Sox47-human GJB2 (SEQ ID NO: 9) Plasmid Construction and Viral Packaging

The Sox47-specific promoter (SEQ ID NO: 1) was constructed into the pAAV-EGFP and pAAV-human GJB2 plasmids to generate the AAV-Sox47-EGFP and AAV-Sox47-human GJB2 (SEQ ID NO: 9) viral packaging plasmids. These plasmids were co-transfected into HEK-293T cells in appropriate amounts together with the AAV-ie capsid protein plasmid and the pHelper plasmid. AAV virions were purified by iodixanol gradient centrifugation, and the viral titer was determined to be 1E12^{~}1E13 GC/mL. The sequence of human GJB2 is available under NCBI accession number: 2706.

### 4.2 Sox47 Promoter-Driven GJB2 Expression in SCs Has No Effect on Hearing

Neonatal C57/BL6 mice on postnatal day 2 (P2) were injected with the viruses AAV-Sox47-EGFP and AAV-Sox47-human GJB2 via round window injection. One month later, auditory brainstem response (ABR) audiometry was performed. It was observed that there was no difference in hearing between the AAV-Sox47-human GJB2 group and the control group (Fig. 5), indicating that the supporting cell (SC)-specific expression driven by the Sox47 promoter has no effect on hearing. Compared with the CAG promoter in Example 1, the Sox47 promoter is a safe promoter.

### Example 5: Sox47 Promoter-Driven GJB2 Re-expression in SCs of GJB2 Conditional Knockout Mice (Sox9Cre;GJB2Loxp) and Its Effects on Hearing

### 5.1 AAV-Sox47-EGFP and AAV-Sox47-human GJB2 Plasmid Construction and Viral Packaging

It's the same as Example 4.1.

### 5.2 Sox47 Promoter-Driven GJB2 Re-expression in Sox9Cre;GJB2Loxp Mice

Neonatal Sox9Cre;GJB2Loxp mice on postnatal day 3 were injected with the viruses AAV-Sox47-EGFP and AAV-Sox47-human GJB2 via round window injection. Fourteen days later, immunofluorescence staining was performed. It was observed that GJB2 was not expressed in supporting cells (SCs) in the AAV-Sox47-EGFP group, whereas GJB2 was re-expressed in the AAV-Sox47-human GJB2 group (Fig. 6).

### 5.3 Sox47 Promoter-Driven GJB2 Re-expression in Sox9Cre;GJB2Loxp Mice Leads to Partial Recovery of Hearing

Sox9Cre;GJB2Loxp mice are generated via the Cre-Loxp system, wherein GJB2 is knocked out specifically in Sox9-labeled supporting cells. Neonatal Sox9Cre;GJB2Loxp mice on postnatal day 3 (P3) were injected with the viruses AAV-Sox47-EGFP and AAV-Sox47-human GJB2 via round window injection. On postnatal day 30 (P30), ABR (auditory brainstem response) audiometry was performed. It was observed that in the experimental group, following the re-expression of GJB2 driven by the Sox47 promoter, the mice exhibited partial recovery of hearing (Fig. 7).

### Sequences

SEQ ID NO: 1: Sox47 promoter
SEQ ID NO: 2: Enhancer
SEQ ID NO: 3: Core SHH promoter element
SEQ ID NO: 4: mNeonGreen DNA sequence
SEQ ID NO: 5: mNeonGreen amino acid sequence
SEQ ID NO: 6: AAV-CAG-nls-EGFP plasmid sequence
SEQ ID NO: 7: AAV-CAG-nls-EGFP-mouse GJB2 plasmid sequence
SEQ ID NO: 8: AAV-Sox47-mNeonGreen plasmid sequence
SEQ ID NO: 9: AAV-Sox47-human GJB2 plasmid sequence

## Claims

1. A cochlear supporting cell-specific enhancer, comprising the sequence set forth in SEQ ID NO: 2 or a homologous sequence having at least 90% sequence identity thereto and retaining the function of SEQ ID NO: 2,
preferably, the homologous sequence is a sequence derived from a mammal, preferably derived from a primate, and more preferably derived from Hominidae.

2. A cochlear supporting cell-specific promoter, comprising:
(1) the sequence set forth in SEQ ID NO: 2 or a sequence having at least 90% sequence identity thereto and retaining the function of SEQ ID NO: 2, and
(2) a core promoter element,
preferably, the core promoter element according to (2) is the promoter of the SHH gene,
preferably, the core promoter element according to (2) has the sequence shown in SEQ ID NO: 3 or a homologous sequence having at least 90% sequence identity thereto,
more preferably, the homologous sequence is the promoter sequence of the SHH gene derived from a mammal, preferably derived from a primate, and more preferably derived from Hominidae.

3. A nucleic acid construct, comprising the cochlear supporting cell-specific promoter according to claim 2,
preferably, the nucleic acid construct further comprises a gene of interest, which is operably linked to the cochlear supporting cell-specific promoter, and/or the nucleic acid construct is a vector,
more preferably, the gene of interest is selected from the group consisting of deafness genes, transcription factor genes, signaling pathway genes, and tool genes that modulate the genes of cochlear supporting cells or that are expressed in cochlear supporting cells.

4. A rAAV vector system, comprising an AAV vector and nucleic acid constructs encoding an AAV virus-required gene, wherein the AAV vector comprises the cochlear supporting cell-specific promoter according to claim 2,
preferably, the AAV vector further comprises a gene of interest, which is operably linked to the cochlear supporting cell-specific promoter.

5. A rAAV virion, comprising the cochlear supporting cell-specific promoter according to claim 2.

6. A host cell, comprising or having integrated into its genome: the cochlear supporting cell-specific promoter according to claim 2, the nucleic acid construct according to claim 3, or the AAV vector system according to claim 4.

7. A pharmaceutical composition, comprising pharmaceutically acceptable excipients, and one or more selected from the group consisting of: the cochlear supporting cell-specific promoter according to claim 2, the nucleic acid construct according to claim 3, the AAV vector system according to claim 4, the rAAV virion according to claim 5, and the host cell according to claim 6.

8. A method for expressing a gene in cochlear supporting cells, comprising the step of introducing a nucleic acid construct comprising the gene into cochlear supporting cells, wherein the gene is operably linked to the cochlear supporting cell-specific promoter according to claim 2.

9. Use of a sequence in a chimeric promoter, the sequence is shown in SEQ ID NO: 2 or is a homologous sequence having at least 90% sequence identity thereto and retaining the function of SEQ ID NO: 2.

10. A method for preparing a chimeric promoter, comprising the step of operably linking (1) the sequence shown in SEQ ID NO: 2 or a sequence having at least 90% sequence identity thereto and retaining the function of SEQ ID NO: 2 with (2) a core promoter element.

11. Use of the cochlear supporting cell-specific promoter according to claim 2 in the preparation of a medicament for treating diseases caused by genetic defects in cochlear supporting cells, wherein the medicament is used for expressing the defective gene in cochlear supporting cells,
preferably, the disease is hereditary deafness caused by genetic defects in cochlear supporting cells,
more preferably, the disease is hereditary deafness caused by the loss-of-function of GJB2.

12. A method for inducing cochlear supporting cells to differentiate into hair cells, comprising the step of expressing transcription factor genes and/or signaling pathway genes associated with hair cell regeneration in cochlear supporting cells, wherein said genes are operably linked to the cochlear supporting cell-specific promoter according to claim 2,
preferably, the method comprises the step of introducing a nucleic acid construct or vector comprising the coding sequences of said transcription factor genes and/or signaling pathway genes into cochlear supporting cells, wherein the coding sequences are operably linked to the cochlear supporting cell-specific promoter.
